# EUROPÄISCHE PATENTANMELDUNG

(11) **EP 2 623 136 A2**
(43) Veröffentlichungstag der Anmeldung: **07.08.2013**
(21) Anmeldenummer: 13152660.0
(22) Anmeldetag: 25.01.2013
(51) Int. Cl.: A61L 31/04, A61L 31/14, A61L 15/22, A61L 15/28

(54) **In der Permeabilität variierbare Membranschicht auf Basis von Carrageen oder Chitosan als Wundauflage**

(30) Priorität: 01.02.2012 DE 102012201463
(71) Anmelder: BSN Medical GmbH, 20253 Hamburg (DE)
(72) Erfinder: KULICKE, Werner-Michael, 22523 Hamburg (DE); KÖSTER, Marina, 23566 Lübeck (DE)
(74) Vertreter: UEXKÜLL & STOLBERG

(57) **Zusammenfassung**

Die Erfindung betrifft ein Verfahren zur Herstellung einer Membranschicht aus vernetztem Polysaccharid, bei dem a) eine angesäuerte Mischung von Polysaccharid mit Weichmacher hergestellt wird, wobei das Polysaccharid ausgewählt ist aus Carrageenen, Chitosanen und Mischungen davon, b) das Polysaccharid in der angesäuerten Mischung mit Polyphosphat vernetzt wird, und c) die Membranschicht gebildet wird. Ferner betrifft die Erfindung entsprechende Membranschichten und die Membranschichten umfassende Wundauflagen. Chitosan ist als Polysaccharid besonders bevorzugt.

Erfindungsgemäße Membranschichten und Wundauflagen werden in Verfahren zur Spalthautentnahme oder zur Behandlung von Problemwunden wie Brandwunden oder chronischen Wunden (beispielsweise *Dekubitus* oder *Ulcus Cruris)* eingesetzt. Die Membranschichten zeichnen sich dadurch aus, dass sie bei der Anwendung nicht zum Kräuseln und zum Quellen neigen.

## Beschreibung

Die vorliegende Erfindung betrifft neuartige vernetzte Polysaccharide, die in Form einer Membranschicht vorliegen. Darüber hinaus betrifft die Erfindung ein Verfahren zur Herstellung dieser Membranschichten. Die Membranschichten werden in Wundauflagen für Problemwunden und für die Spalthautgewinnung eingesetzt.

Im Stand der Technik sind Wundauflagen für Problemwunden bekannt. Die Unterschiedlichkeit von verschiedenen Problemwunden stellt hohe Anforderungen an die Wundauflagen. Bei stark exudierenden Wunden wie Brandwunden muss die aus der Wunde austretende Flüssigkeit aufgenommen oder abgeleitet werden, wobei ein Flüssigkeitsfilm auf der Wunde verbleiben soll. Bei weniger stark exudierenden Wunden wie beispielsweise chronischen Wunden ist dagegen eine geringere Permeabilität der Wundauflage wünschenswert.

Beispielsweise offenbart die DE 10 2004 047 115 A Wundauflagen mit einer permeablen Polymermatrix. Zu deren Herstellung wird eine mit einem Weichmacher versetzte Polymerlösung zu einer Wundauflage weiterverarbeitet. Alternativ erfolgt die Herstellung der Wundauflage, indem zunächst eine Polymermembran hergestellt und diese anschließend in eine Lösung des Weichmachers überführt wird. In beiden Fällen führt der Weichmacher zu einer Erhöhung der Permeabilität der Wundauflage, wobei die Wasserdampfdurchlässigkeit von der Menge des zugefügten Weichmachers abhängt.

Beispielhafte Polymere gemäß DE 10 2004 047 115 A sind Chitosane. Chitosane, d.h. Polymere vom Typ der Poly-(1,4)-2-amido-2-desoxy-β-D-Glucose, sind deacetyliertes Chitin. Sie sind antimikrobiell, geruchsadsorbierend und filmbildend, wirken stimulierend auf die Wundheilung, besitzen ein Quellvermögen, rufen bei entsprechender Reinheit keine allergischen Reaktionen hervor, sind für Warmblüter nicht toxisch und können biologisch abgebaut werden. Demzufolge sind Chitosane für Wundauflagen grundsätzlich gut geeignet.

Als nachteilig hat sich jedoch herausgestellt, dass bekannte für Wundauflagen für Problemwunden eingesetzte Membranen (beispielsweise auf der Basis von Chitosanen) für eine eher trockene Applikation nicht geeignet sind, u.a. weil sie zum Kräuseln, d.h. zum Hochbiegen der Ränder neigen. Außerdem quellen die Wundauflagen der DE 10 2004 047 115 A beim Gebrauch stark an. In einem späten Stadium der Wundheilung ist jedoch eine hohe Adsorption von Feuchtigkeit aus der Wunde, verbunden mit Kräuseln und Quellen der Wundauflage, unerwünscht. Stattdessen soll eine gewisse Feuchtigkeit auf der Wunde beibehalten, überflüssige Feuchtigkeit jedoch abgeführt werden.

Es hat sich nun herausgestellt, dass diese Probleme des Standes der Technik dadurch überwunden werden können, dass eine Membranschicht eingesetzt wird, die aus einer angesäuerten Mischung von Polysaccharid und Weichmacher und Vernetzen der Mischung mit Polyphosphat herstellbar ist.

Die Erfindung betrifft demzufolge in einem ersten Aspekt ein Verfahren zur Herstellung einer Membranschicht aus vernetztem Polysaccharid, bei dem:
a) eine angesäuerte Mischung von Polysaccharid mit Weichmacher hergestellt wird, wobei das Polysaccharid ausgewählt ist aus Carrageenen, Chitosanen und Mischungen davon,
b) das Polysaccharid in der angesäuerten Mischung mit Polyphosphat vernetzt wird, und
c) die Membranschicht gebildet wird.

Mit dem erfindungsgemäßen Verfahren ist es möglich, die Vorteile von Chitosanen und Carrageenen bei der Wundheilung, einschließlich der Hämostase, noch besser zu nutzen. Zudem bleibt die äußere Oberfläche der erfindungsgemäßen Membranschicht nach Kontakt mit einer feuchten Wunde elastisch und trocken. Die der Wunde zugewandte Seite erhält durch partielle Adsorption des Wundsekrets eine gallertartige Konsistenz. Dies führt sowohl zu einer Wundreinigung als auch zu einer Anpassung der Membran an die Unebenheiten einer Wundoberfläche. Das physiologisch wichtige Feuchtmilieu der Wunde wird hierdurch während der exudativen Heilungsphase beibehalten.

Wie in der DE 10 2004 047 115 A bereits beschrieben ist, kann eine erfindungsgemäße Membranschicht über eine Porenstruktur verfügen, die durch vorübergehende Einlagerungen in die Polymermatrix erhältlich ist. Eine solche Membranschicht hat insbesondere den Vorteil, dass die auszubildenden Poren größenmäßig gut definierbar sind. So können beispielsweise Makroporen durch Anlagerungen von Silikatpartikeln und Mikroporen durch Einlagerungen von Polyethylenglykol mit einer Molmasse von 1500 bis 100.000 g/mol ausgebildet werden. Silikatpartikel oder Polyethylenglykol werden im Anschluss an die Herstellung der Membranschicht wieder aus der Polymermatrix herausgelöst. Auf diese Weise kann eine Porengröße von beispielsweise 0,1 bis 100 µm eingestellt werden. Darüber hinaus kann die Oberflächenstruktur der Wundauflage bei der Wundheilung eine Rolle spielen, sodass es auch hierfür vorteilhaft ist, eine bestimmte Porenstruktur vorzusehen.

Die erfindungsgemäßen Membranschichten zeichnen sich ferner dadurch aus, dass sie beim Kontakt mit der Wunde nicht zum Kräuseln oder Rollen neigen. Darüber hinaus zeigen die erfindungsgemäßen Wundauflagen eine geringe Neigung zum Quellen. Während bei einer Vernetzung beispielsweise mit Glutaraldehyd, wie es in der DE 10 2004 047 115 A vorgeschlagen wird, eine Quellung der Membranschichten um 300 bis 500 % beobachtet wird (bezogen auf das Gewicht), quellen erfindungsgemäße Membranschichten, die mit Polyphosphat vernetzt sind, um weniger als 100 Gew.-%, beispielsweise weniger als 80 Gew.-%, wie weniger als 50 Gew.-%

In einer bevorzugten Ausführungsform ist das Polysaccharid ein Chitosan. Bevorzugte Chitosane besitzen einen Deacetylierungsgrad von 70 % oder mehr, vorzugsweise 80 % oder mehr, insbesondere 85 % oder mehr, wie insbesondere 90 % oder mehr (bestimmt durch ¹H-NMR- und ¹³C-NMR-Spektroskopie).

Dabei ist bevorzugt, dass das Chitosan eine mittlere Molmasse von 150.000 g/mol oder mehr und eine breite Molmassenverteilung im Bereich von 150.000 bis 700.000 g/mol besitzt, detektierbar durch SEC/MALLS-DRI (size-exclusion chromatography with multi-angle laser light scattering-differential refractive index detection).

Carrageene sind langkettige Kohlenhydrate, die in den Zellen von Rotalgenarten vorkommen. Es handelt sich um lineare, anionische Hydrokolloide. Vorzugsweise werden Carrageene vom Typ λ, κ und 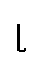 eingesetzt, sowie Mischungen derselben.

Bei dem für die Vernetzung eingesetzten Polyphosphat handelt es sich vorzugsweise um Natriumpolyphosphat der Formel (NaPO₃)ₓ. Die Konzentration des Polyphosphats in der für die Vernetzung typischerweise eingesetzten wässrigen Polyphosphatlösung beträgt vorzugsweise 0,5 bis 20 Gew.-%, bevorzugter 1 bis 10 Gew.-%, wie 4 bis 8 Gew.-%, beispielsweise etwa 6 Gew.%.

Weiterhin ist bevorzugt, dass der Weichmacher ausgewählt ist aus Glycerin, Zitronensäureestern, Glyceroltriacetaten, 1,2-Propandiol und Polyethylenglycol mit einer Molmasse bis 400 g/mol. Besonders bevorzugt als Weichmacher ist Glycerin.

Ferner ist bevorzugt, dass die in Schritt a) hergestellte angesäuerte Mischung eine wässrige Lösung ist und die Konzentration des Weichmachers in der Lösung 0,2 bis 5 Gew.-%, vorzugsweise 0,5 bis 3 Gew.-%, insbesondere 2 bis 3 Gew.-% beträgt.

Die in Schritt a) hergestellte angesäuerte Mischung ist vorzugsweise eine wässrige Lösung, und die Konzentration des Polysaccharids in der Lösung beträgt vorzugsweise 0,2 bis 5 Gew.-%, vorzugsweise 0,5 bis 3 Gew.-%, insbesondere 2 bis 3 Gew.-%.

Bei Schritt a) wird vorzugsweise so vorgegangen, dass aus einem der genannten Polysaccharide (mit geeigneter Molmasse und Molmassenverteilung) eine Lösung in Wasser hergestellt wird. Zu dieser Lösung wird - wie in der DE 10 2004 047 115 A beschrieben - eine verdünnte organische oder anorganische Säure gegeben, z.B. Zitronensäure, Weinsäure, Ameisensäure, Salzsäure, Phosphorsäure, Milchsäure oder Essigsäure, vorzugsweise Milchsäure oder Essigsäure, insbesondere Milchsäure. Zu dieser Polysaccharid-Säure-Lösung in Wasser wird dann der Weichmacher gegeben.

In Schritt a) wird dann vorzugsweise so vorgegangen, dass die angesäuerte Mischung filtriert und in einer gewünschten Dicke auf eine Oberfläche gerakelt wird, beispielsweise in einer Dicke von 0,05 - 1 mm.

In Schritt b) des erfindungsgemäßen Verfahrens wird vorzugsweise so vorgegangen, dass die Mischung mit einer wässrigen Lösung von Polyphosphat in Kontakt gebracht und dadurch vernetzt wird. Gegebenenfalls wird nach dem Vernetzen in Schritt b) weiterer Weichmacher zugegeben.

Nach dem Vernetzen des Polysaccharids in der angesäuerten Mischung wird in Schritt c) die Membranschicht gebildet, beispielsweise indem getrocknet wird. Die Trocknung erfolgt vorzugsweise in einem Umluft- oder IR-Ofen oder -trockner, beispielsweise in einem Trockenkanal mit Umluft- und/oder IR-Trockner. Die Geschwindigkeit der Trocknung in dem Trockenkanal hängt von der Dicke der Membranschicht und der Trocknungstemperatur/Intensität ab.

In einer bevorzugten Ausführungsform wird das vernetzte Polymer nach Schritt b) mit Wasser gespült und dann in Schritt c) getrocknet.

Bei dem erfindungsgemäßen Verfahren kann im Anschluss an die Schritte a) und c) gegebenenfalls so vorgegangen werden, dass die Membranschicht in weiteren Schritten
d) sterilisiert und/oder
e) zur besseren Handhabbarkeit zwischen Polymerfolien gelagert wird.

Es ist dabei bevorzugt, dass die Membranschicht in Schritt d) mittels Wasserdampf oder durch Gamma-Strahlung sterilisiert wird. Eine Sterilisierung mit Ethylenoxid ist möglich (jedoch nicht wenn Glycerin als Weichmacher eingesetzt wird).

Letztendlich ist es bevorzugt, dass die Membranschicht für eine bessere Applikation und Handhabung zwischen zwei Polymerfolien, beispielsweise Polyethylenfolien, aufbewahrt wird. Weil erfindungsgemäße Membranschichten eine ausreichende Festigkeit aufweisen, ist es nicht notwendig, dass die Wundauflage mehrschichtig aufgebaut ist und einen Träger umfasst. In einer bevorzugten Ausführungsform besteht die Wundauflage demzufolge aus der Membranschicht.

Außerdem betrifft die Erfindung eine Membranschicht, die nach dem erfindungsgemäßen Verfahren herstellbar ist.

In einem weiteren Aspekt betrifft die Erfindung eine aus vernetztem Polysaccharid hergestellte Membranschicht, die
a) eine Zugfestigkeit von 0,5 N/cm oder mehr, vorzugsweise 0,1 N/cm oder mehr, insbesondere 2 N/cm oder mehr, wie 5 N/cm oder mehr, jeweils bestimmt gemäß EN ISO 527 Teil 3,
b) eine Wasserdampfdurchlässigkeit (WVTR), bestimmt gemäß DIN EN 13726-2:2002 bei Kontakt mit Wasserdampf, von 300 g/(m²·24 h) oder mehr, und
c) eine Dicke, bestimmt gemäß DIN 53370, von 1 mm oder weniger besitzt,
wobei das Polysaccharid ausgewählt ist aus Carrageenen, Chitosanen und Mischungen davon.

Diese Membranschicht ist vorzugsweise nach dem erfindungsgemäßen Verfahren hergestellt.

Die Wasserdampfdurchlässigkeit beträgt vorzugsweise 1500 bis 6000 g/(m²·24 h) bei Kontakt mit Wasserdampf und 10.000 g/(m²·24 h) oder mehr bei Kontakt mit Flüssigkeit, jeweils bestimmt gemäß DIN EN 13726-2:2002.

Obwohl es grundsätzlich möglich ist, dass die Membran oder eine andere Schicht der Wundauflage einen oder mehrere pharmazeutische Wirkstoffe enthält, ist in allen Ausführungsformen der Erfindung bevorzugt, dass die Membran keinen pharmazeutischen Wirkstoff enthält. Besonders bevorzugt enthält die gesamte Wundauflage keinerlei pharmazeutische Wirkstoffe.

Eine bevorzugte Dicke der Membranschicht beträgt 15 bis 80 µm, bestimmt gemäß DIN 53370. Besonders bevorzugt ist eine Dicke von 40 bis 60 µm.

In einer bevorzugten Ausführungsform ist die Membranschicht transparent oder transluzent. Dies bedeutet, dass die Lichtdurchlässigkeit über 70 % beträgt, bestimmt gemäß ASTM D 1003 (Total Light %). Dadurch kann die Wundheilung beobachtet und kontrolliert werden.

In einer weiteren bevorzugten Ausführungsform beträgt die Wasseradsorption der Membranschicht, bestimmt gemäß DIN EN 13726-1:2002, weniger als 0,1 g/(10 cm²·24 h).

In einem weiteren Aspekt betrifft die Erfindung eine Wundauflage, die eine erfindungsgemäße Membranschicht umfasst. Wie erwähnt ist es möglich, dass die Wundauflage lediglich aus der erfindungsgemäßen Membranschicht besteht und (nur zur Lagerung) zur besseren Haltbarkeit zwischen Polymerfolien gelagert wird.

Es ist alternativ möglich, dass die Wundauflage den folgenden mehrschichtigen Aufbau besitzt:
a) die Membranschicht aus vernetztem Polysaccharid auf der Wundkontaktseite,
b) eine perforierte Membran aus hydrophilem oder hydrophobem Material,
c) eine Superabsorberschicht,
d) eine Adsorptions- oder Absorptionsschicht, vorzugsweise aus Polyurethan, und
e) eine bakterienundurchlässige Schicht (Bakterienbarriereschicht).

In dieser Ausführungsform ist es bevorzugt, die Schicht d) antimikrobiell auszurüsten, beispielsweise mit Chlorhexidin, Silber und/oder Polyhexamethylenbiguanid (PHMB).

Weiter alternativ ist es möglich, dass die Wundauflage den nachfolgenden mehrschichtigen Aufbau besitzt:
a) die Membranschicht aus vernetztem Polysaccharid auf der Wundkontaktseite und
b) eine Vliesschicht aus Cellulose, Viskose und/oder Polyurethan, die gegebenenfalls mit Polypropylen und/oder Polyethylen gemischt sein kann.

Letztendlich ist es möglich, dass die Wundauflage den folgenden mehrschichtigen Aufbau besitzt:
a) eine Silikon(schaum)schicht auf der Wundkontaktseite und
b) die Membranschicht aus vernetztem Polysaccharid.

Bei einem mehrschichtigen Aufbau ist eine Perforierung möglich.

In allen Ausführungsformen der Erfindung ist es möglich und bevorzugt, dass zusätzlich zu der Membranschicht aus vernetztem Polysaccharid eine Schicht vorhanden ist, die antimikrobiell ausgerüstet ist, beispielsweise mit Chlorhexidin, Silber und/oder PHMB.

In einer weiteren Ausführungsform betrifft die Erfindung die Membranschicht oder die Wundauflage zur Verwendung in einem Verfahren zur Spalthautentnahme oder zur Behandlung von Problemwunden, insbesondere Brandwunden oder chronischen Wunden wie *Dekubitus* oder *Ulcus cruris.*

Ein Dekubitus ist eine durch langanhaltenden Druck ausgelöste Minderdurchblutung der Hautschichten mit anschließender Schädigung des Gewebes bis zum Knochen. Er entsteht vor Allem über Knochenvorsprüngen und ist eine Sekundärerkrankung, die in Folge von Immobilität bei gleichzeitig prädisponierenden Faktoren entsteht. Derartige Druckgeschwüre (durch Druck erzeugte ischämische Hautläsionen) entstehen vor allem, wenn der Auflagedruck der harten Matratze oder einer Sitzfläche den Wert von 25 mmHg für mehr als 2 Stunden überschreitet (vgl. Seiler, 2002; Gerhard Schröder; Pflege von Menschen mit chronischen Wunden, 2. Auflage 2010; Verlang Hans Huber, Bern).

Als Ulkus (Geschwür) wird ein tiefer Gewebedefekt definiert, der mindestens bis in die Dermis (Unterhaut) reicht und im Gegensatz zu einer Erosion (Schürfwunde) immer mit einer Narbe abheilen wird. Cruris bezeichnet die Lokalisation am Unterschenkel, sodass mit dem Begriff *Ulcus cruris* das Auftreten eines Ulkus im Unterschenkelbereich beschrieben wird (vgl. Joachim Dissemond; Pflege von Menschen mit chronischen Wunden, 2. Auflage 2010; Verlag Hans Huber, Bern).

Spalthaut eignet sich insbesondere zum Decken großer Flächen, z.B. nach Verbrennungen, zumal sich die Spenderstellen in kurzer Zeit regenerieren können. Die Entnahme erfolgt üblicherweise an ebenen Hautarealen, bevorzugt am Oberschenkel, Rücken, Gesäß oder Bauch. Bei der Gewinnung von Spalthaut werden, nach Unterspritzung mit Lokalanästhetikum, die obersten Epithelschichten flächenhaft mit dem Skalpell oder Dermatom abgetragen (vgl. Peter D. Asmussen, Brigitte Söllner; Wundmanagement: Prinzipien und Praxis, Hippokrates Verlag GmbH, Stuttgart 1995).

Die Vorteile der Erfindung ergeben sich insbesondere aus dem folgenden Anwendungsbeispiel. Dabei beziehen sich - wenn nichts Anderes angegeben ist - alle Prozentangaben auf das Gewicht.

Zur Herstellung der Membran wird in einem ersten Schritt ein Chitosan geeigneter Molmasse und Molmassenverteilung bereitgestellt. Mit Hilfe der Molmasse wird die erwünschte Viskosität der Chitosanlösung eingestellt, die auf das Rakeln der Lösung (vergleiche Schritt b) sowie auf die Entstehung der Blasen beim Lösungsvorgang einen entscheidenden Einfluss hat. Mit einer Molmassenverteilung von geeigneter Breite können die mechanischen Eigenschaften der Membranen (Elastizität, Reißdehnung, Anschmiegsamkeit auf der Haut) eingestellt werden.

Mit dem gewählten Chitosan oder Chitosangemisch wird dann eine 2-3%ige Chitosansuspension in destilliertem Wasser angesetzt. Zu dem so vorgequollenen Chitosan wird eine Menge Milchsäure dazugegeben, um eine 2,5%ige Milchsäurelösung zu erhalten. Die vollständige Lösungszeit hängt von dem Anteil Chitosan ab und beträgt bis etwa 2 Stunden. In die Chitosanlaktatlösung wird außerdem eine bestimmte Menge Glycerin gegeben, die bis 3% der Lösung ausmachen kann. Dabei bedeutet eine 3%ige Glycerin-lösung in einer 3%igen Chitosanlösung ein Gewichtsverhältnis von Chitosan zu Glycerin von 1:1. Mit dem Glycerinanteil können die mechanischen Eigenschaften der Membranen und auch die Durchlässigkeit für verschiedene Gase und Wasserdampf eingestellt werden, wie in der DE 10 2004 047 115 A beschrieben ist.

Die so erhaltene Chitosanlaktat-Glycerin-Lösung wird dann filtriert und in unterschiedlichen Dicken von 0,05 bis 1 mm auf einer Polycarbonat- oder einer Plexiglas-Platte gerakelt. Die Rakeldicke ist nach oben nicht begrenzt. Um die kleineren Dicken zu erhalten, muss die Lösung gleichmäßig aufgesprüht werden.

Nachdem der Polymerfilm auf der Platte völlig blasenfrei gemacht wurde, wird die Platte mit dem nicht getrockneten Polymerfilm in einem dritten Schritt in eine Wanne mit Polyphosphatlösung unterschiedlicher Konzentration, abhängig vom Chitosan (sowie dessen Konzentration), Rakeldicke sowie gewünschter Vernetzungsgeschwindigkeit gegeben, wobei nun entweder durch eine Symplex-Reaktion oder eine ionotrope Gelbildung die Vernetzung zu einer vollständig ausgebildeten Membran erfolgte.

Nach der vollständigen Vernetzung wird die Platte in einem vierten Schritt aus dem Polyphosphatbad genommen. Die Membran wird mit destilliertem Wasser mehrfach gespült, leicht vorgetrocknet, mit wasserfreiem Glycerin von beiden Seiten bestrichen (oder in ein Glycerinbad eingelegt) und dann mit Heißluft getrocknet.

Die Membran kann für eine bessere Applikation und Handhabung zwischen zwei Polyethylenfolien aufbewahrt werden. Sie kann gegebenenfalls mittels Wasserdampf oder durch Gamma-Strahlung sterilisiert werden. Eine Sterilisierung mit Ethylenoxid ist möglich (jedoch nicht, wenn Glycerin als Weichmacher eingesetzt wird).

## Patentansprüche

1. Verfahren zur Herstellung einer Membranschicht aus vernetztem Polysaccharid, bei dem:
a) eine angesäuerte Mischung von Polysaccharid mit Weichmacher hergestellt wird, wobei das Polysaccharid ausgewählt ist aus Carrageenen, Chitosanen und Mischungen davon,
b) das Polysaccharid in der angesäuerten Mischung mit Polyphosphat vernetzt wird, und
c) die Membranschicht gebildet wird.

2. Verfahren nach Anspruch 1, **dadurch gekennzeichnet, dass** das Polysaccharid ein Chitosan ist.

3. Verfahren nach Anspruch 2, **dadurch gekennzeichnet, dass** das Chitosan einen Deacetylierungsgrad von 70 % oder mehr besitzt, vorzugsweise 80 % oder mehr, insbesondere 85 % oder mehr, wie insbesondere 90 % oder mehr.

4. Verfahren nach Anspruch 2 oder 3, **dadurch gekennzeichnet, dass** das Chitosan eine mittlere Molmasse von 150.000 g/mol oder mehr und eine breite Molmassenverteilung im Bereich von 150.000 bis 700.000 g/mol besitzt.

5. Verfahren nach einem der vorhergehenden Ansprüche, **dadurch gekennzeichnet, dass** der Weichmacher ausgewählt ist aus Glycerin, Zitronensäureestern, Glyceroltriacetaten, 1,2-Propandiol und Polyethylenglykol mit einer Molmasse bis 400 g/mol, wobei insbesondere Glycerin als Weichmacher bevorzugt ist.

6. Verfahren nach einem der vorhergehenden Ansprüche, **dadurch gekennzeichnet, dass** die in Schritt a) hergestellte angesäuerte Mischung eine wässrige Lösung ist und die Konzentrationen des Weichmachers in der Lösung 0,2 bis 5 Gew.-%, vorzugsweise 0,5 bis 3 Gew.-%, insbesondere 2 bis 3 Gew.-% beträgt.

7. Verfahren nach einem der vorhergehenden Ansprüche, **dadurch gekennzeichnet, dass** die in Schritt a) hergestellte angesäuerte Mischung eine wässrige Lösung ist und die Konzentration des Polysaccharids in der Lösung 0,2 bis 5 Gew.-%, vorzugsweise 0,5 bis 3 Gew.-%, insbesondere 2 bis 3 Gew.-% beträgt.

8. Membranschicht, herstellbar nach dem Verfahren gemäß einem der vorhergehenden Ansprüche.

9. Membranschicht, die aus vernetztem Polysaccharid hergestellt ist und die
a) eine Zugfestigkeit von 0,5 N/cm oder mehr, vorzugsweise 0,1 N/cm oder mehr, insbesondere 2 N/cm oder mehr, wie 5 N/cm oder mehr, jeweils bestimmt gemäß EN ISO 527 Teil 3,
b) eine Wasserdampfdurchlässigkeit (WVTR), bestimmt gemäß DIN EN 13726-2:2002 bei Kontakt mit Wasserdampf, von 300 g/(m²·24 h) oder mehr, und
c) eine Dicke, bestimmt gemäß DIN 53370, von 1 mm oder weniger besitzt,
wobei das Polysaccharid ausgewählt ist aus Carrageenen, Chitosanen und Mischungen davon.

10. Membranschicht gemäß Anspruch 9, die hergestellt ist gemäß dem Verfahren eines der Ansprüche 1 bis 7.

11. Membranschicht nach einem der Ansprüche 8 bis 10, **dadurch gekennzeichnet, dass** die Wasserdampfdurchlässigkeit 1500 bis 6000 g/(m²·24 h) bei Kontakt mit Wasserdampf und 10.000 g/(m²·24 h) oder mehr bei Kontakt mit Flüssigkeit beträgt (jeweils bestimmt gemäß DIN EN 13726-2:2002).

12. Membranschicht nach einem der Ansprüche 8 bis 11, **dadurch gekennzeichnet, dass** sie keinen pharmazeutischen Wirkstoff enthält.

13. Membranschicht nach einem der Ansprüche 8 bis 12, **dadurch gekennzeichnet, dass** sie eine Dicke, bestimmt gemäß DIN 53370, von 15 bis 80 µm, vorzugsweise 40 bis 60 µm besitzt.

14. Membranschicht nach einem der Ansprüche 8 bis 13, **dadurch gekennzeichnet, dass** sie transparent oder transluzent ist und vorzugsweise eine Lichtdurchlässigkeit, bestimmt gemäß ASTM D 1003 (Total Light %), von über 70 % aufweist.

15. Membranschicht nach einem der Ansprüche 8 bis 14, **dadurch gekennzeichnet, dass** die Wasseradsorption, bestimmt gemäß DIN EN 13726-1:2002, weniger als 0,1 g/(10 cm²· 24 h) beträgt.

16. Wundauflage, die eine Membranschicht gemäß einem der Ansprüche 8 bis 15 umfasst.

17. Wundauflage gemäß Anspruch 16, **dadurch gekennzeichnet, dass** sie den folgenden mehrschichtigen Aufbau besitzt:
a) die Membranschicht aus vernetztem Polysaccharid auf der Wundkontaktseite,
b) eine perforierte Membran aus hydrophilem oder hydrophobem Material,
c) eine Superabsorberschicht,
d) eine Adsorptions- oder Absorptionsschicht, vorzugsweise aus Polyurethan, und
e) eine Bakterienbarriereschicht.

18. Wundauflage gemäß Anspruch 16, **dadurch gekennzeichnet, dass** sie den folgenden mehrschichtigen Aufbau besitzt:
a) die Membranschicht aus vernetztem Polysaccharid auf der Wundkontaktseite und
b) eine Vliesschicht aus Cellulose, Viskose und/oder Polyurethan, die gegebenenfalls mit Polypropylen und/oder Polyethylen gemischt sein kann.

19. Wundauflage gemäß Anspruch 16, die den folgenden mehrschichtigen Aufbau besitzt:
d) eine Silikon(schaum)schicht auf der Wundkontaktseite und
e) die Membranschicht aus vernetztem Polysaccharid.

20. Membranschicht gemäß einem der Ansprüche 8 bis 15, oder Wundauflage gemäß einem der Ansprüche 16 bis 19, zur Verwendung in einem Verfahren zur Spalthautentnahme oder zur Behandlung von Problemwunden, insbesondere Brandwunden oder chronischen Wunden wie *Dekubitus* oder *Ulcus cruris.*
